# EUROPEAN PATENT SPECIFICATION

(11) **EP 0 359 428 B1**
(45) Date of publication and mention of the grant of the patent: **09.08.1995**
(21) Application number: 89308681.9
(22) Date of filing: 25.08.1989
(51) Int. Cl.: C07K 1/107, A61K 47/00, G01N 33/543

(54) **Protein derivatives and a process for their preparation**
Protein-Derivate und Verfahren zu ihrer Herstellung
Dérivés de protéines et leur procédé de préparation

(30) Priority: 26.08.1988 GB 8820378
(43) Date of publication of application: 21.03.1990
(73) Proprietor: Offord, Robin Ewart, 1257 Croix-de-Rozon (CH); Rose, Keith, CH-1208 Geneva (CH)
(72) Inventor: Offord, Robin Ewart, 1257 Croix-de-Rozon (CH); Rose, Keith, CH-1208 Geneva (CH)
(74) Representative: Hallybone, Huw George

(56) References cited:
- WO-A-85/05638
- H. PEETERS: "Protides of the biological fluids", 1986, pages 35-38, Pergamon Press; R.E. OFFORD et al.: "Press-stud protein conjugates"
- PEPTIDES 1986 - PROCEEDINGS OF THE 19TH EUROPEAN PEPTIDE SYMPOSIUM, Porto Carras, Chalkidiki, 31st August - 5th September 1986, pages 219-222, Walter de Gruyter & Co., Berlin, DE; K. ROSE et al.: "Enzyme-assisted semisynthesis of polypeptide active esters for subsequent spontaneous coupling"
- PEPTIDES 1986 - PROCEEDINGS OF THE 19TH EUROPEAN PEPTIDE SYMPOSIUM, Porto Carras, Chalkidiki, 31st August - 5th September 1986, pages 279-281, Walter de Gruyter & Co., Berlin, DE; R.E. OFFORD et al.: "Press-stud protein conjugates"
- BIOCHEMICAL JOURNAL, vol. 249, 1988, pages 83-88, GB; K. ROSE et al.: "Enzyme-assistes semisynthesis of polypeptide active esters and their use"

## Description

### Field of the Invention

This invention relates to a process for the preparation of protein derivatives and protein conjugates which are of use in medicine. More particularly the invention relates to the preparation of hydrazide derivatives of proteins by enzyme catalysed reverse proteolysis.

### Background to the Invention

There is considerable interest in medicine in the use of protein conjugates, for either therapy or diagnosis. The conjugate generally comprises a protein, such as an antibody, to which is attached an effector or a reporter group, for example a cytotoxic agent or a group capable of being radioactively labelled.

In general, attachment of effector or reporter groups to proteins is made by group-specific, but not regio-specific reactions (e.g. by iodination of tyrosine residues, or acylation of lysine residues). As a result, even mono-reacted material is in general itself heterogeneous, being a mixture of species each with one group attached to one of several residues of the type involved in the modification reaction.

For a protein conjugate intended for use in medicine, much is to be gained from the homogeneity of the product of site specific attachment of a group of interest, in contrast to the product obtained by the non-regiospecific procedures described above. Thus, the advantages of site-specific modification of antibodies of the IgG type have been reported [Murayama, A et al, Immunochemistry 15, 523-528, (1978); Rodwell, J.D et al Proc. Natl. Acad. Sci. USA 83, 2632-2636, (1986) and European Patent Specification No. 88695]. In this case, regiospecificity was achieved by oxidation of the carbohydrate of IgG molecules, followed by reductive alkylation. The success of such an approach depends upon the existence of appropriate glycosylation, so it is not generally applicable to other classes of polypeptide, and since even some IgG molecules posses more than one site of glycosylation, the approach may not always be site specific. International Patent Application WO85/05638 describes the non-regiospecific chemical derivatisation of enzymes with hydrazides.

The specificity of proteases working in reverse has been exploited to fix activating groups exclusively at the carboxyl terminus of polypeptides [Offord, R.E. and Rose, K. in Protides of the Biological Fluids (H. Peeters, ed.) Pergamon, Oxford pp35-38, (1986); Offord, R.E. et al in Peptides 1986 (D. Theodoropoulos, ed.) W. de Gruyter, Berlin, pp279-281, (1986); Rose, K et al in Peptides 1986 (ibid), pp219-222, (1986); Rose, K. et al, Biochem. J. 249, 83-88, (1988); and European Patent Specification No. 243929]. In a subsequent chemical reaction an effector or reporter group may be directed specifically to the activated group of the modified polypeptide. (It is not usually possible or desirable to fix enzymatically the group of interest directly to the polypeptide chain for reasons of enzyme specificity and the large excess of amino component generally required to drive the enzymatic coupling to high yield). The specificity of the conjugation reaction between the effector or reporter group and the activated polypeptide may be achieved by chemical complementarity of the reacting groups in the effector or reporter molecule and the polypeptide, or, in the case of the effector or reporter molecule being itself a polypeptide fragment, by conformational assistance.

Activating groups which are aromatic aldehydes, aliphatic aldehydes or aromatic amines have been described for use in protease directed reactions of the above type. Conjugation was achieved via formation and reduction of a Schiff base (reductive alkylation) and specificity was obtained by virtue of the relative reactivities of aromatic versus aliphatic amines at low pH.

We have now found that it is possible to use enzyme catalysed reverse proteolysis to attach hydrazide groups specifically to the carboxyl termini of proteins. No side chain protection is required and the coupling advantageously takes place under very mild conditions and in aqueous solutions where denaturation of the protein can be kept to a minimum. Hydrazide groups are not normally found in proteins and site specific conjugations may be effected by directing appropriately modified effector or reporter groups to the hydrazide group when these latter have been attached enzymically to the protein. The resulting conjugate requires no further treatment, in contrast to previous reports of site specific attachment (Murayama, A et al, Rodwell, J.D et al, Offord, R.E. and Rose, K., Offord, R.E. et al, ibid) where reductive stabilisation of a Schiff base is necessary. Since such reduction can be responsible for the irreversible formation of unwanted inter- and intra-molecular links, higher specificity may thus be obtained using conjugation via hydrazone formation.

Non-specific attachment of hydrazide groups to proteins using chemical reagents has previously been described, and protein conjugates have been formed via hydrazone formation [King, T.P. et al, Biochemistry 25, 5774-5779 (1986)].

### Summary of the Invention

According to one aspect of the invention we provide a process for the preparation of a compound of formula (1): 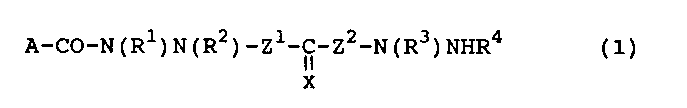
wherein
A-CO- is the residue of a protein or peptide A-CO₂H, wherein -CO₂H is the C-terminal carboxyl group of the protein or peptide;
R¹, R², R³ and R⁴, which may be the same or different, is each a hydrogen atom or an alkyl, aryl or aralkyl group; Z¹ and Z², which may be the same or different, and which may be present or absent, is each a spacer group;
X is an oxygen or sulphur atom; and the salts thereof which comprises condensing a compound of formula (2):

A-CO₂H (2)

or an activated derivative thereof with a compound of formula (3): 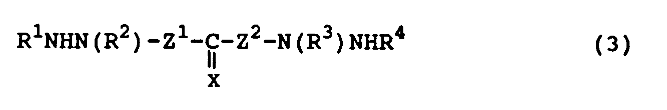
in the presence of an enzyme capable of catalyzing the formation of a peptide bond.

In the compounds of formula (1), the residue of a protein or peptide ACO₂H -presented by A-CO- may be the residue of any naturally occurring, synthetic or semi-synthetic protein or peptide, including proteins and peptides produced by hybridoma and recombinant DNA techniques. Particular examples include residues of plasma proteins, such as immunoglobulins, (for example polyclonal, monoclonal and recombinant antibodies and fragments thereof), fibrinogen, albumin and transferrin, hormones, for example steroid hormones such as oestrogens, insulin, erythropoietin and somatotropin, lymphokines such as interleukin 1 or interleukin 2, cytokines such as tumour necrosis factor, industrially and therapeutically useful enzymes such as tissue plasminogen activator and enzyme inhibitors such as tissue inhibitor of metalloproteinases.

When the group R¹, R², R³ or R⁴, in compounds of formula (1) is an alkyl group it may be for example a C₁₋₆alkyl group such as a methyl or ethyl group. Examples of aryl groups represented by R¹, R², R³ or R⁴ are C₆₋₁₂aryl groups, for example phenyl groups. Aralkyl groups represented by R¹, R², R³ or R⁴ include ArC₁₋₃ alkyl, e.g. phenC₁₋₃alkyl such as benzyl or phenethyl.

When Z¹ or Z² in compounds of formula (1) is a spacer group it may be for example an optionally substituted aliphatic hydrocarbyl chain optionally interrupted by one or more heteroatoms selected from -O-or -S- or one or more -N(R⁵)- (where R⁵ is a hydrogen atom or a C₁₋₆alkyl group), cycloaliphatic or aromatic groups.

Thus, in particular, Z¹ or Z² may represent an optionally substituted straight or branched C₁₋₁₀alkylene, C₂₋₁₀alkenylene or C₂₋₁₀alkynylene chain, optionally interrupted by one or more heteroatoms selected from -O- or -S- or one or more -N(R⁵) [e.g. -NH- or -N(CH₃)-] cycloaliphatic (e.g. C₃₋₈ cycloalkylene such as cyclohexylene) or aromatic (e.g. C₆₋₁₂arylene, such as phenylene) groups.

Particular examples of Z¹ or Z² include -CH₂-, -(CH₂)₂-, -CH₂OCH₂-, -CH₂SCH₂, -(CH₂)₃- or -(CH₂)₄.

Salts of compounds of formula (1) include salts with acids and bases, for example acid addition salts such as hydrochlorides or hydrobromides, or alkali metal salts such as sodium or potassium salts.

A-CO- in compounds of formula (1) is the residue of a protein or peptide A-CO₂H wherein -CO₂H is the C-terminal carboxyl group of the protein or peptide.

A preferred group of compounds of formula (1) are those wherein A-CO- is the residue of a plasma protein such as fibrinogen or an immunoglobulin or a fragment thereof, a hormone, such as insulin, a cytokine such as tumour necrosis factor, or a therapeutically useful enzyme such as tissue plasminogen acitvator.

In particular, A-CO- is preferably the residue of an immunoglobulin or a fragment thereof. The immunoglobulin or immunoglobulin fragment may in general belong to any immunoglubulin class. Thus, for example, it may be an antibody belonging to an immunoglobulin M class or, in particular, an antibody belonging to an immunoglobulin G class. The antibody, or fragment thereof, may be of animal, for example mammalian origin and may be for example of murine, rat or human origin. It may be a natural antibody or a fragment thereof, or, if desired, a recombinant antibody or antibody fragment, i.e. an antibody or antibody fragment which has been produced using recombinant DNA techniques.

Particular recombinant antibodies or antibody fragments include, (1) those having an antigen binding site at least part of which is derived from a different antibody, for example those in which the hypervariable or complementarity determining regions of one antibody have been grafted into the variable framework regions of a second, different antibody (as described in European Patent Specification No. 239400); (2) recombinant antibodies or fragments wherein non-Fv sequences have been substituted by non-Fv sequences from other, different antibodies (as described in European Patent Specifications Nos. 171496, 173494 and 194276); or (3) recombinant antibodies or fragments possessing substantially the structure of a natural immunoglobulin but wherein the hinge region has a different number of cysteine residues from that found in the natural immunoglobulin, or wherein one or more cysteine residues in a surface pocket of the recombinant antibody or fragment is in the place of another amino acid residue present in the natural immunoglobulin (as described in International Patent Application Nos. PCT/GB 88/00730 and PCT/GB 88/00729 respectively).

The antibody or antibody fragment may be of polyclonal, or, preferably, monoclonal origin. It may be specific for any number of antigenic determinants, but is preferably specific for one. The antigenic determinant may be any hapten or an antigenic determinant associated with animals, e.g. humans, [for example normal animal tissue or organ cell-associated antigens, tumour cell-associated antigens (for example oncofetal antigens such as carcinoembryonic antigen or alphafetoprotein, placental antigens such as chorionic gonadotropin and placental alkaline phosphatase, and prostate antigens such as prostatic acid phosphatase and prostate specific antigen) and antigens associated with components of body fluids such as fibrin or platelets], viruses, bacteria and fungi.

Antibody fragments include for example fragments derived by proteolytic cleavage of a whole antibody, such as F(ab')₂, Fab' or Fab fragments, or fragments obtained by recombinant DNA techniques, for example Fv fragments (as described in International Patent Application No. PCT/GB 88/00747).

R¹, R², R³ and R⁴ in compounds of formula (1) are preferably hydrogen atoms or alkyl, especially methyl groups. Compounds of formula (1) in which R¹, R², R³ and R⁴ is each a hydrogen atom are particularly preferred.

A further generally preferred group of compounds of formula (1) are those in which Z¹ and Z² are both absent or are both present and are the same.

When Z¹ and Z² in compounds of formula (1) are present, they are preferably C₁₋₁₀alkylene chains, optionally interrupted by one or more -O- or -S- atoms or -N(R⁵)- groups.

X in compounds of formula (1) is preferably an oxygen atom.

A particularly useful group of compounds according to the invention has the formula (1a): 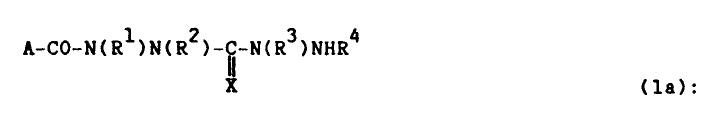
wherein A-CO-, R¹, R², R³, R⁴ and X are as defined for formula (1) and the salts thereof.

Especially useful compounds of this type have the formula (1b): 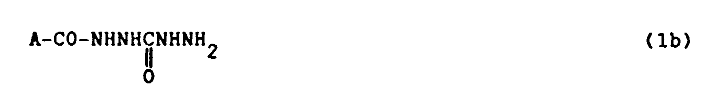
wherein A-CO- is as defined for formula (1) and the salts thereof.

Compounds of formulae (1a) and (1b) wherein A-CO- is the residue of a protein or peptide A-CO₂H wherein -CO₂H is the C-terminal carboxyl group of the protein or peptide are particularly preferred. Compounds of this type wherein A-CO- is the residue of an immunoglobulin or a fragment thereof are especially useful.

Compounds of formula (1) may be prepared by the following processes wherein A-CO-, R¹, R², R³, R⁴, Z¹ Z² and X are as defined for formula (1) except where otherwise specified.

Thus we provide a process for the preparation of a compound of formula (1) which comprises condensing a compound of formula (2):

A-CO₂H (2)

or an activated derivative thereof with a compound of formula (3): 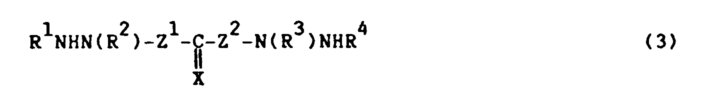
in a solvent, for example an aqueous solvent, at a suitable temperature, for example around ambient temperature.

Where it is desired to prepare a compound of formula (1) where Z¹ and Z² are not the same it may be necessary to protect the group -N(R³)NHR⁴ prior to the reaction. Conventional protection procedures may be used, and the group may be deprotected once the compound of formula (1) has been obtained, using standard deprotection techniques.

Suitable enzymes for use in the invention include proteases, in particular endopeptidases, such as serine proteinases, e.g. trypsin or chymotrypsin, or lysyl endopeptidase, or exopeptidases, for example carboxypeptidases, such as carboxypeptidase Y.

In general, the reaction may be performed under advantageously mild conditions selected to favour the condensation of the compound of formula (2) with the compound of formula (3). The exact conditions used will depend on the enzyme in use. Thus, for example, using trypsin as the enzyme the reaction may be effected in the presence of a solvent, especially an aqueous solvent, for example water or an aqueous organic solvent, for example aqueous dimethylsulphoxide or aqueous butane-1,4-diol, at an appropriate pH, for example in the range pH4-8, at a suitable temperature, for example around ambient temperature.

Other enzymes may be employed in the process using the same or similar conditions, the exact nature of which may be determined empirically using appropriate small-scale tests in accordance with conventional practice prior to carrying out the condensation reaction.

Salts of compounds of formula (1) may be prepared using standard procedures, for example by reacting a compound of formula (1) with an acid or base in an aqueous solvent.

Proteins or peptides of formula (2) are readily available or may be obtained using standard procedures.

Intermediates of formula (3) are either known compounds or may be prepared from known starting materials using methods analogous to those used for the preparation of the known compounds.

Compounds of formula (1) are useful for the preparation of protein conjugates in which a protein is linked to a further molecule of interest, for example another protein or a peptide, or an effector, or reporter molecule, the linkage comprising a -N(R¹)N(R²) -Z¹C(=X)Z²-N(R³)N= group.

In conjugates of this type the groups R¹, R², R³, Z¹, Z² and X are as defined for formula (1), and the various preferences expressed for certain types of these groups in connection with formula (1) are also to be understood to apply to protein conjugates according to this aspect of the invention.

Thus, the present invention provides for the preparation of a compound of formula (4): 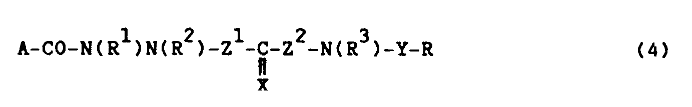
wherein A-CO-, R¹, R², R³, Z¹, Z² and X is as defined for formula (1), R is a protein or a peptide or an effector or reporter group and Y is a linking group formed by coupling a -NHR⁴ group where R⁴ is as defined for formula (1) with a reactive group in the group R, comprising preparation of a compound of formula (1) according to the first aspect of the invention and coupling said compound of formula (1) with a protein or a peptide or an effector or reporter group.

Particular examples of compounds of formula (4) include compounds of formula (5): 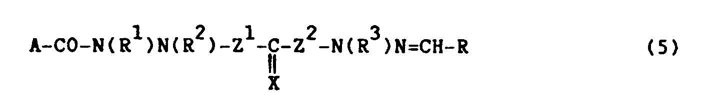
wherein A-CO-, R¹, R², R³, Z¹, Z², X and R are as just defined.

In compounds of formulae (4) and (5), R may be for example a protein or peptide as defined above for A-CO-, or an effector or a reporter group. Effector groups include, for example any physiologically active substance, antibacterial, antiviral, or antifungal compound. Particular physiologically active substances include antineoplastic agents, including cytotoxic and cytostatic agents, toxins, hormones, anti-inflammatory compounds, and substances active as cardiovascular, e.g. fibrinolytic, and central nervous system, agents. Reporter groups include for example a group which is detectable by analytical means in vitro and/or in vivo, such as a group capable of being radioactively labelled, a fluorescent group or a group capable of being monitored by NMR or ESR spectroscopy.

It will be appreciated that in the formation of the compounds of formulae (4) and (5) the starting material R must possess a group capable of reacting with the hydrazide group of the compound of formula (1). Such groups may be readily identified, and include, especially, aldehyde groups. The appropriate group may already be present in the starting material or, when necessary, may be introduced using conventional techniques for example as described in the Examples herein.

### Brief Description of the Drawing

The invention is further illustrated in the following non-limiting examples and with reference to the accompanying drawing in which

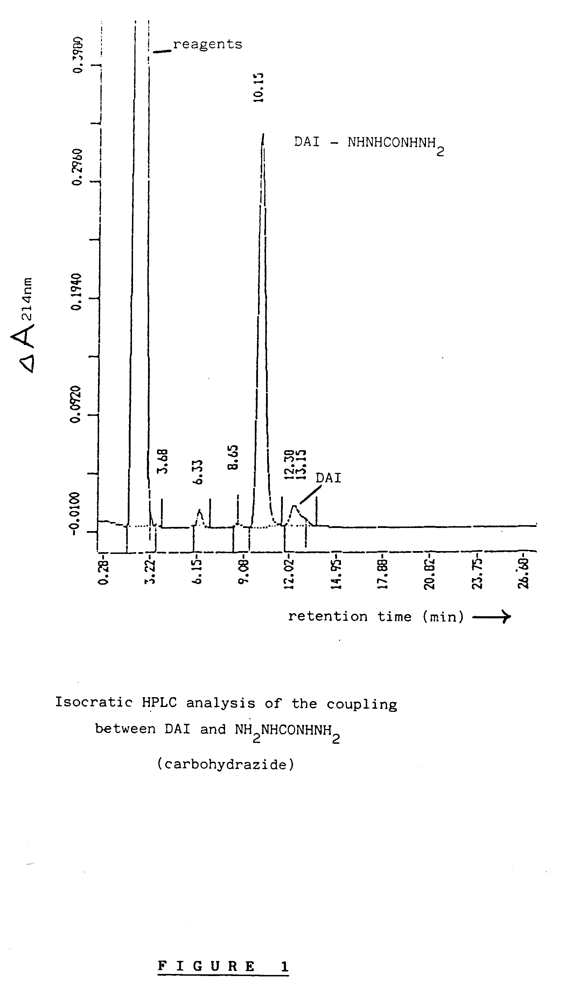

Figure 1 shows the result of isocratic HPLC analysis of a sample obtained from the reaction of des (ala^{B30}) insulin (DAI) and 1,1-carbonyldihydrazide in the presence of lysyl endopeptidase, and indicates the separation of DAI-NHNHCONHNH₂, a compound made according to the invention, from the DAI starting material.

### Description of Specific Embodiments

The following Examples illustrate the invention:

### Example 1

### Coupling of 1,1-carbonyldihydrazide to DesAla^{B30} insulin (DAI)-Preparation of DAI-NHNHCONHNH₂

Des(Ala^{B30}) insulin (DAI) was prepared as previously described (Morihara et al, Biochem. Biophys. Res. Commun. 92 1980 396-402). To 1,1-carbonyldihydrazide (180mg - Fluka) was added 2ml distilled water followed by 2ml dimethylsulphoxide (Fluka puriss.) and 12»l acetic acid (Fluka puriss.). The apparent pH was measured using a glass electrode (model GK2421 C, Radiometer, Copenhagen) after calibration with aqueous standards (Merck), no correction being applied. The pH was lowered to a measured 5.5 by adding approximately 12.5»l 98-100% formic acid (Merck). DAI (30mg) was dissolved in 1.5ml of the above solution and then 0.5mg Lysyl Endopeptidase (from Achromobacter; Wako GmbH) was added in 30»l water. Analytical, followed by preparative, HPLC was performed on System 1:
System 1: Beckman Gold system. The column was Machery Nagel Nucleosil 300A 5»m C8 25cm x 4mm i.d. operated at 0.6ml/minute. Solvent A was 0.3M ammonium sulphate and solvent B was 0.3M ammonium sulphate in 35% acetonitrile. The two solvents were made up using a stock solution of 3M ammonium sulphate which had been adjusted to an indicated pH of 2.7 (glass electrode) by careful addition of concentrated sulphuric acid. Detection was at 214nm for analytical runs and 254nm for preparative runs.

Fig. 1 shows the result of isocratic HPLC analysis on System 1. DAI is cleanly transformed into a more hydrophilic (earlier-eluting) compound (identified as DAI-NHNHCONHNH₂, see below) in about 90% yield. Preparative separation was achieved under the same conditions, injecting the sample in batches corresponding to 5mg of original DAI, with manual collection of fractions. After dilution with water, protein was recovered on a C18 SepPak (Waters) used according to the manufacturer's instructions (methanol wash, equilibration with 0.1% trifluoroacetic acid (TFA), sample application after prior dilution with an equal volume of water, washing- with 0.1% TFA/20% acetonitrile, elution with 0.1% TFA/40% acetonitrile. Solvents were removed in a vacuum centrifuge (SpeedVac Concentrator, Savant) with the heater switched off.

The product, DAI-NHNHCONHNH₂, was characterised by electrophoresis, peptide mapping, reversed phase HPLC, and by specific conjugation with 2,4-dihydroxybenzaldehyde. On electrophoresis on cellulose acetate at pH 8 (system described by Rose et al, J. Chromatogr. 210 1981 301-309) the product behaved as an insulin derivative having lost a single negative charge (compare Rose et al, Biochem. J. 211 1983 671-676). This is as expected for the structure, since the carboxyl group of Lys^{B29} of DAI has become a hydrazide group, which is uncharged at pH 8. On digestion using a protease isolated from Armillaria mellea which cleaves on the N-terminal side of Lys residues (see e.g. Rose et al, in Peptides 1984, Ragnarsson, U. ed., pp235-238, Almqvist and Wiksell International, Stockholm, 1984), des(Lys^{B29}-Ala^{B30}) insulin was liberated (identified by HPLC and electrophoresis on cellulose acetate by comparison with authentic material) along with a small fragment having the expected properties (on high voltage paper electrophoresis) of Lys-Ala-NHNHCONHNH₂. Upon reversed phase HPLC in System 1 (Fig. 1) the DAI-NHNHCONHNH₂ eluted earlier than DAI or native insulin (DAI and native insulin coelute on the system), consistent with the product having an extra positive charge at low pH. In view of the above data and the method of synthesis (compare Rose et al, Biochem. J. 211 1983 671-676), the product has the expected structure DAI-NHNHCONHNH₂, with the hydrazide group attached through the carboxyl group of Lys^{B29}.

Zinc-free porcine insulin (control) and DAI-NHNHCONHNH₂ were incubated separately at 100»m concentration in 0.1M sodium acetate buffer at pH 4.6 at 22^{o}C in the presence and absence of 500»M 2,4-dihydroxybenzaldehyde (Fluka). At intervals, 10»l aliquots were analysed by HPLC. Results clearly showed that insulin did not react with the aldehyde, that DAI-NHNHCONHNH₂ was stable in the absence of aldehyde, that the aldehyde was stable and that DAI-NHNHCONHNH₂ reacted cleanly to produce a single more hydrophobic conjugate. Under similar conditions, DAI-NHNHCONHNH₂ reacted with 4-carboxybenzaldehyde (Fluka) to give a product more hydrophobic on HPLC and possessing one more negative charge (than DAI-NHNHCONHNH₂) on electrophoresis at pH 8, whereas no reaction occurred with unmodified insulin.

### Example 2

### Reaction of DAI-NHNHCONHNH₂ with HCO-CO-ferrioxamine labelled with ⁵⁵Fe.

In 0.2ml 0.1M sodium acetate buffer pH4.6, DAI-NHNHCONHNH₂ (prepared and purified as in Example 1) was incubated, at 22^{o}C at a concentration of 100»M, with 560»M HCO-CO-ferrioxamine (commerical deferoxamine was acylated with Boc-L-SerONsu, deprotected, then labelled with approximately 10⁶dpm of ⁵⁵Fe per 112nmol and then oxidised to the HCO-CO-form using periodate in ethylene glycol; the preparation was diluted to required specific activity). After 24h, the sample was subjected to gel filtration on a Sephadex G50 fine column in 0.1M sodium acetate buffer followed by liquid scintillation counting of all fractions. Results showed close to the expected incorporation of radioactivity into the protein fraction (14.3% of the radioacitivty was incorporated; in view of the excess ferrioxamine reagent, the theoretical maxiumum incoporation would be 17.8%). A control incubation of labelled HCO-CO-ferrioxamine with unmodified insulin showed no incorporation of radioactivity into the protein fraction.

### Example 3

### Coupling of 1,1-carbonyldihydrazide to des(pentapeptide^{B26-30}) insulin using chymotrypsin.

1,1-Carbonyldihydrazide was coupled to des(pentapeptide^{B26-B30}) insulin (DPI; prepared by known procedure involving mild peptic digestion of porcine insulin, and purification by ion exchange chromatography on DEAE A25 in Tris buffer containing urea) using alpha-chymotrypsin (Sigma Chemical Co.) under the conditions of Example 1 except that the scale was 5mg and the reaction time was 24hours. Analysis by HPLC showed clean conversion (in about 62% yield) to an earlier-eluting product which was identified as the expected DPI-NHNHCONHNH₂ by electrophoresis on cellulose acetate and by its ability to bind to a gel having covalently bound aldehyde groups (unmodified insulin did not bind to the aldehyde column).

### Example 4

### Production of a carbonyldihydrazide derivative of IgG corresponding in size to a F(ab′)₂ fragment.

To 0.2ml of a solution of IgG (10mg/ml in phosphate-buffered saline) was added 0.8ml of a solution of 1,1-carbonyldihydrazide (2.5M, pH5.5: made by dissolving 50g of the carbonyldihydrazide in 210ml of water, adding 1.2ml glacial acetic acid followed by 1.5ml formic acid, and making up to 220ml with water). Solid trypsin (porcine, 2mg) was added and the mixture allowed to stand at 20^{o}C. Electrophoresis of small samples in a standard SDS-acrylamide gel system showed that there was a progressive digestion of the IgG. A fragment that had the apparent molecular mass of a F(ab′)₂ molecule was predominant between roughly 24 hours and 72 hours of digestion. Little IgG was left after 72 hours. The modified fragments were isolated from the trypsin by gel filtration on a 25 x 0.8cm column (f.p.l.c.) of Superose 12. The column buffer was obtained by diluting the 2.5M carbonyldihydrazide solution to 2M with water. The flow rate was 0.5ml/minute.

The peak corresponding to F(ab′)₂ was almost completely separated from that of trypsin, and was recycled on the same column in the same buffer to complete the removal of the enzyme. The column buffer was then changed to 0.1M NH₄HCO₃ and 1ml of 2mg/ml solution of soyabean trypsin inhibitor passed through the system. The carbonyldihydrazide was then removed from the F(ab′)₂-like material on this column.

The product of a 24 hour digestion under the above conditions was isolated as above and shown to have an incorporation of hydrazide of 0.06 moles/mole IgG.

### Example 5

### Production of a Fab-like IgG-ferrioxamine conjugate

To a sample of IgG antibody (0.5 ml, 23.3 mg/ml in phosphate buffered saline, known to give Fab-like fragments on trypsin digestion) was added 50 »l trypsin (porcine, TPCK-treated 15 mg/ml in 10-³M HCl) and the mixture was left for 8 hours at room temperature. A further 100 »l of trypsin solution was then added, and the digest allowed to continue for a further 21.5 hours. There was a white precipitate at this point. Solid 1,1-carbonyldihydrazide (155mg) was added to the digest and after 1 hour and 20 minutes the reaction was stopped by the addition of 200 »l of pancreatic trypsin inhibitor (Bayer, 13 mg/ml).

After an appropriate work-up and coupling to aldehydic ⁵⁵Ferrioxamine (prepared as described in Example 2) it was found that approximately 20% of the fab-like IgG molecules were labelled. About 90% of this radioactivity was removable in a subsequent trypsin digest, illustrating that the labelling took place substantially at trypsin-sensitive sites at the C-terminus.

## Claims

1. A process for the preparation of a compound of formula (1): 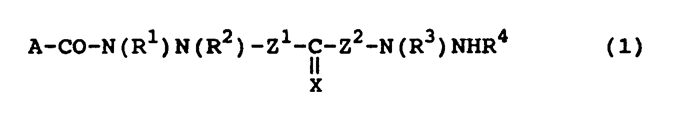 wherein
A-CO- is the residue of a protein or peptide A-CO₂H, wherein -CO₂H is the C-terminal carboxyl group of the protein or peptide;
R¹, R², R³ and R⁴, which may be the same or different, is each a hydrogen atom or an alkyl, aryl or aralkyl group; Z¹ and Z², which may be the same or different, and which may be present or absent, is each a spacer group;
X is an oxygen or sulphur atom; and the salts thereof which comprises condensing a compound of formula (2):
A-CO₂H (2)
or an activated derivative thereof with a compound of formula (3): 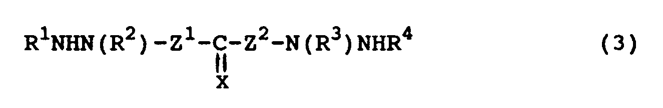 in the presence of an enzyme capable of catalyzing the formation of a peptide bond.

2. A process according to claim 1 wherein Z¹ and Z² are absent.

3. A process according to claim 1 or 2 wherein R¹, R², R³ and R⁴ is each a hydrogen atom.

4. A process according to any of the preceding claims wherein X is an oxygen atom.

5. A process according to any of the preceding claims wherein A-CO is the residue of an immunoglobulin or a fragment thereof.

6. A process according to any one of the preceding claims wherein R¹, R², R³ and R⁴ is each hydrogen, Z¹ and Z² are absent and X is an oxygen atom.

7. A process for the preparation of a compound of formula (4): 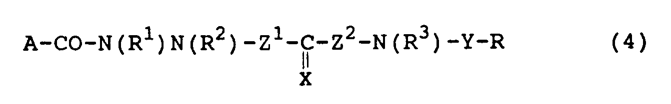 wherein A-CO, R¹, R², R³, Z¹, Z² and X are as defined in any one of claims 1 to 6
R is a protein or a peptide or an effector or reporter group, and
Y is a linking group formed by coupling the -NHR⁴ group of formula (1) (wherein R⁴ is a hydrogen atom or an alkyl, aryl or aralkyl group) with reactive group in the group R,
comprising preparation of a compound of formula (1): 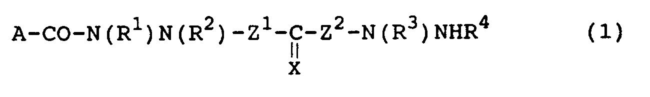 according to any one of claims 1 to 6 and coupling said compound of formula (1) with a protein or peptide or an effector or reporter group possessing a group capable of reacting with the group -NHR⁴.

8. A process according to claim 7 wherein the protein or the peptide or the effector or reporter group possesses an aldehyde group.

## Patentansprüche

1. Ein Verfahren zur Herstellung einer Verbindung der Formel (1) 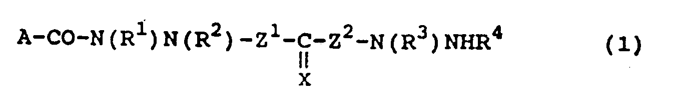 worin A-CO- für den Rest eines Proteins oder Peptids A-CO₂H steht, in welchem -CO₂H die C-terminale Carboxylgruppe des Proteins oder Peptids ist;
R¹, R², R³ und R⁴, die gleich oder verschieden sein können, jeweils für ein Wasserstoffatom oder eine Alkyl-, Aryl- oder Aralkylgruppe stehen;
Z¹ und Z², die gleich oder verschieden und anwesend oder abwesend sein können, jeweils eine Abstandsgruppe darstellen;
X für ein Sauerstoff- oder Schwefelatom steht;
und der Salze derselben,
bei welchem Verfahren eine Verbindung der Formel (2):
A-CO₂H (2)
oder ein aktiviertes Derivat derselben mit einer Verbindung der Formel (3) 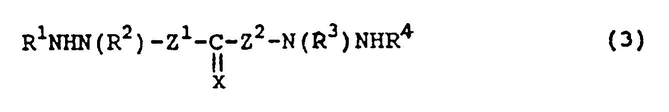 in Gegenwart eines Enzyms, das zur Katalysierung der Bildung einer Peptidbindung befähigt ist, kondensiert wird.

2. Ein verfahren nach Anspruch 1, bei welchem Z¹ und Z² fehlen.

3. Ein Verfahren nach Anspruch 1 oder 2, bei welchem R¹, R², R³ und R⁴ jeweils für ein Wasserstoffatom stehen.

4. Ein Verfahren nach einem der vorhergehenden Ansprüche, bei welchem X für ein Sauerstoffatom steht.

5. Ein Verfahren nach einem der vorhergehenden Ansprüche, bei welchem A-CO der Rest eines Immunglobulins oder eines Fragmentes desselben ist.

6. Ein Verfahren nach einem der vorhergehenden Ansprüche, bei welchem R¹, R², R³ und R⁴ jeweils für Wasserstoff stehen, Z¹ und Z² fehlen und X ein Sauerstoffatom bedeutet.

7. Ein Verfahren zur Herstellung einer Verbindung der Formel (4): 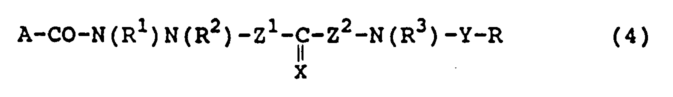 worin A-CO, R¹, R², R³, Z¹, Z² und X die in einem der Ansprüche 1 bis 6 genannte Bedeutung haben,
R ein Protein oder ein Peptid oder eine Effektor- oder Reportergruppe darstellt und
Y eine Verbindungsgruppe bedeutet, die gebildet ist durch Kopplung der Gruppe -NHR⁴ in der Formel (1) (in welcher R⁴ für ein Wasserstoffatom oder eine Alkyl-, Aryl- oder Aralkylgruppe steht) mit einer reaktiven Gruppe in der Gruppe R,
welches Verfahren die Herstellung einer Verbindung der Formel (1): 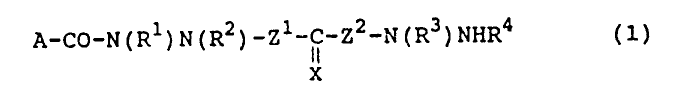 nach einem der Ansprüche 1 bis 6 und die Kopplung der genannten Verbindung der Formel (1) mit einem Protein oder Peptid oder einer Effektor- oder Reportergruppe, die eine zur Reaktion mit der Gruppe -NHR⁴ befähigte Gruppe besitzt, umfaßt.

8. Ein Verfahren nach Anspruch 7, bei welchem das Protein oder das Peptid oder die Effektor- oder Reportergruppe eine Aldehydgruppe besitzt.

## Revendications

1. Procédé pour préparer un composé de formule (1): 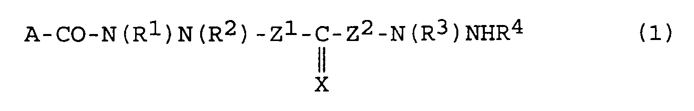 dans laquelle
A-CO- est le résidu d'une protéine ou d'un peptide A-CO₂H, où -CO₂H est le groupe carboxyle du C terminal de la protéine ou du peptide;
R¹, R², R³ et R⁴, qui peuvent être identiques ou différents, sont chacun un atome d'hydrogène ou un groupe alkyle, aryle ou aralkyle;
Z¹ et Z², qui peuvent être identiques ou différents, et qui peuvent être présents ou absents, sont chacun un groupe d'espacement;
X est un atome d'oxygène ou de soufre; et les sels de ce composé,
qui comprend la condensation d'un composé de formule (2):
A-CO₂H (2)
ou d'un dérivé activé de celui-ci avec un composé de formule (3): 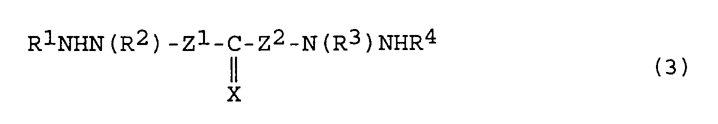 en présence d'une enzyme capable de catalyser la formation d'une liaison peptide.

2. Procédé conforme à la revendication 1, dans lequel Z¹ et Z² sont absents.

3. Procédé conforme à la revendication 1 ou 2, dans lequel R¹, R², R³ et R⁴ sont chacun un atome d'hydrogène.

4. Procédé conforme à l'une quelconque des précédentes revendications, dans lequel X est un atome d'oxygène.

5. Procédé conforme à l'une quelconque des précédentes revendications, dans lequel A-CO est le résidu d'une immunoglobuline ou d'un fragment de celle-ci.

6. Procédé conforme à l'une quelconque des précédentes revendications, dans lequel R¹, R², R³ et R⁴ sont chacun un atome d'hydrogène, Z¹ et Z² sont absents et X est un atome d'oxygène.

7. Procédé pour la préparation d'un composé de formule (4): 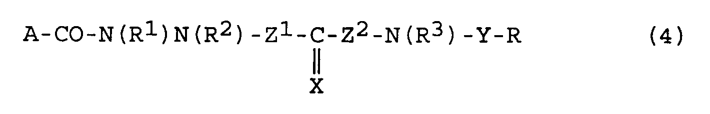 dans laquelle A-CO, R¹, R², R³, Z¹, Z² et X sont tels que définis dans l'une quelconque des revendications 1 à 6
R est une protéine ou un peptide ou un groupe effecteur ou reporteur, et
Y est un groupe de liaison formé en couplant le groupe -NHR⁴ de la formule (1)(dans laquelle R⁴ est un atome d'hydrogène ou un groupe alkyle, aryle ou aralkyle) avec un groupe réactif du groupe R,
comprenant la préparation d'un composé de formule (1): 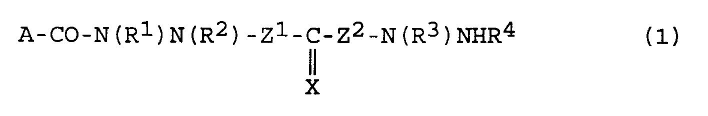 conformément à l'une quelconque des revendications 1 à 6 et le couplage dudit composé de formule (1) avec une protéine ou un peptide ou un groupe effecteur ou reporteur possédant un groupe capable de réagir avec le groupe -NHR⁴.

8. Procédé conforme à la revendication 7, dans lequel la protéine ou le peptide ou le groupe effecteur ou reporteur possède un groupe aldéhyde.
